# EUROPEAN PATENT SPECIFICATION

(11) **EP 1 786 886 B1**
(45) Date of publication and mention of the grant of the patent: **23.02.2011**
(21) Application number: 05808769.3
(22) Date of filing: 01.09.2005
(51) Int. Cl.: C09K 11/06

(54) **ANTHRACENE DERIVATIVES AND ORGANIC LIGHT EMITTING DEVICE USING THE SAME AS A LIGHT EMITTING MATERIAL**
ANTHRACENDERIVATE UND DEREN VERWENDUNG ALS LICHTEMITTIERENDES MATERIAL IN ORGANISCHER LICHTEMITTIERENDER VORRICHTUNG
DERIVES D'ANTHRACENE ET DISPOSITIF ORGANIQUE ELECTROLUMINESCENT LES UTILISANT EN TANT QUE MATERIAU ELECTROLUMINESCENT

(30) Priority: 02.09.2004 KR 2004070100
(43) Date of publication of application: 23.05.2007
(73) Proprietor: LG Chem, Ltd., Youngdungpo-gu Seoul 150-721 (KR)
(72) Inventor: KIM, Ji Eun; 7-403 LG chemistry partner APT.,, Daejeon Metropolitan City 305-340 (KR); SON, Se Hwan; 102-704 Hyundai APT.,, Daejeon Metropolitan City 305-340 (KR); LEE, Jae Chol; 8-107 LG chemistry APT.,, Daejeon Metropolitan City 305-340 (KR); BAE, Jae Soon; 106-305 Expo APT.,, Daejeon Metropolitan City 305-761 (KR); KIM, Kong Kyeom; 107-703 Expo APT.,, Daejeon Metropolitan City 305-761 (KR); KANG, Min Soo; 114-1407 Han Maeul APT.,, Daejeon Metropolitan City 305-756 (KR); JEON, Sang Young;, Seoul 143-849 (KR)
(74) Representative: HOFFMANN EITLE
(86) International application number: PCT/KR2005/002896
(87) International publication number: WO 2006/025700

(56) References cited:
- EP-A1- 0 726 500
- WO-A-2005/100506
- JP-A- 11 249 311
- JP-A- 2004 059 535
- US-A1- 2004 126 617
- US-A1- 2005 113 477

## Description

### Technical Field

The present invention relates to a novel compound and an organic light emitting device using the same. More particularly, the present invention pertains to novel anthracene derivatives having electroluminescence and an organic light emitting device using the same as a light emitting material.

### Background Art

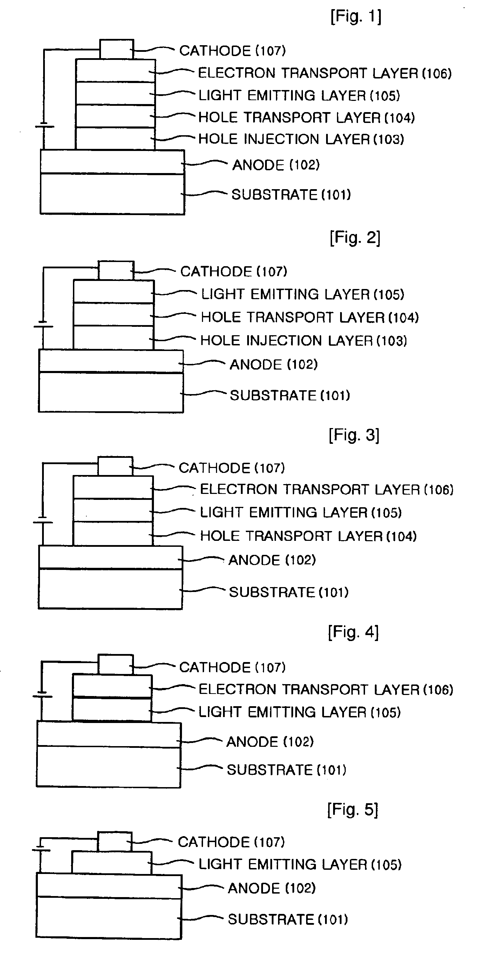

Generally, an organic light emitting device has a structure in which thin organic material layers are layered between two opposite electrodes, and the organic material layers may form a multilayered structure including different materials so as to increase the efficiency and stability of the device. For example, as shown in FIG. 1, the organic light emitting device may have a structure in which a substrate 101, an anode 102, a hole injection layer 103, a hole transport layer 104, a light emitting layer 105, an electron transport layer 106, and a cathode 107 are sequentially layered.

Meanwhile, an effort has been made to use a compound containing an anthracene group in an organic light emitting device since early in the 1960's. In the year 1965, Helfrich and Pope reported the realization of blue organic electroluminescence using a single crystal of anthracene for the first time. However, a high voltage is required to emit light using a single crystal of anthracene, and there are many problems in commercialization due to the short life of the device (W. Helfrich, W. G. Schneider, Phys. Rev. Lett. 14, 229, 1965. M. Pope, H. Kallmann, J. Giachino, J. Chem. Phys., 42, 2540, 1965).

Recently, much effort has been made to introduce various substitutes to an anthracene molecule and apply the resulting molecule to an organic light emitting device. For example, US. Pat. Nos. 5,935,721 (Formula A), 5,972,247 (Formula B), 6,251,531 (Formula C), and 5,635,308 (Formula D), EP 0681019 (Formula E), and Korean Patent Registration No. 10-0422914 (Formula F) disclose anthracene derivatives as a blue light emitting material. Furthermore, EP 1009044 (Formula G) discloses an anthracene derivative as a hole transport material. Additionally, Japanese Patent Laid-Open Publication No. Hei.11-345686 (Formula H) discloses an anthracene derivative as an electron transport material and a blue light emitting material.

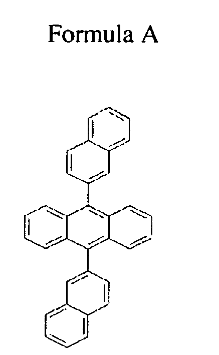

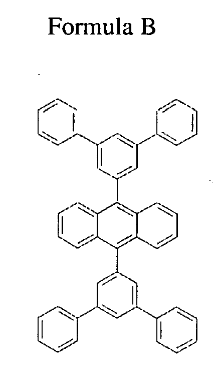

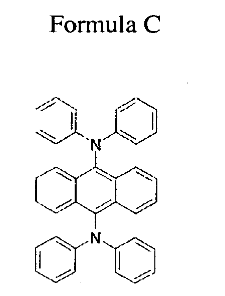

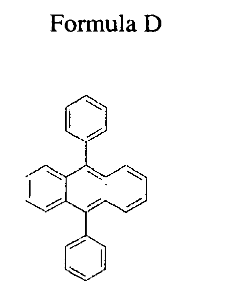

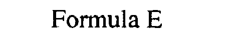

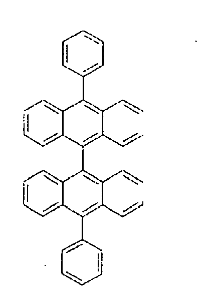

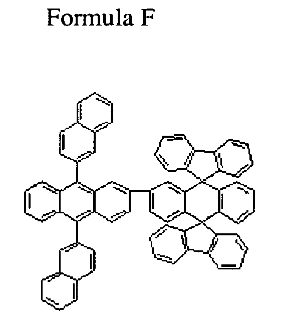

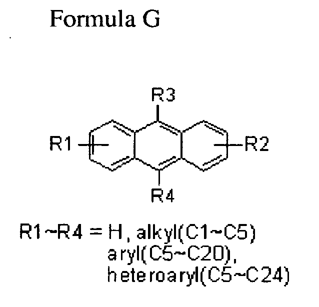

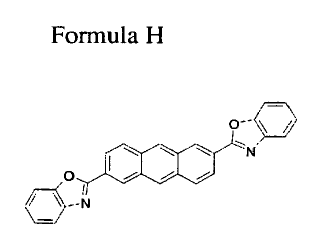

Additionally, Korean Patent Laid-Open Publication No. 10-2002-0003025 discloses an anthracene derivative as an electron transport material, and Korean Patent Registration No. 10-0422914 discloses a compound, in which an aryl group having a high melting point is introduced to a position 2 of anthracene, as a light emitting material.

WO 2005/100506 discloses anthracene derivatives for a light emitting layer.

US 2004/0126617 discloses anthracene derivables for organic electroluminescent devices.

### Disclosure of Invention

### Technical Problem

The present inventors have conducted studies into the synthesis of an anthracene derivative having a novel structure, resulting in the finding that it is possible to improve the life of an organic light emitting device and to realize low voltage driving when this compound is used as a light emitting material of the organic light emitting device.

Therefore, an object of the present invention is to provide an anthracene derivative having a novel structure and an organic light emitting device using the same.

### Technical Solution

The present invention provides a compound having the following Formula 1.

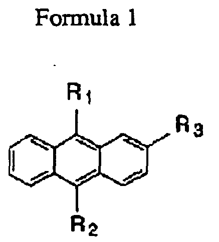

In Formula 1, R1, R2, and R3 are each as defined in the appended Claim 1.

Furthermore, the present invention provides an organic light emitting device which comprises a first electrode, one or more organic material layers, and a second electrode which are sequentially layered. One or more layers of the organic material layers include the compound of Formula 1.

### Brief Description of the Drawings

FIGS. 1 to 5 illustrate examples of organic light emitting devices to which a novel compound according to the present invention is applied.

### Best Mode for Carrying Out the Invention

Hereinafter, a detailed description of the present invention will be given.

As described above, anthracene has high light emitting efficiency, and has been known to be an important chemical structure capable of constituting an organic material layer of an organic light emitting device since the 1960's. Furthermore, many patent documents disclose that it is possible to increase the performance of an organic light emitting device by applying a substitute, particularly an aryl group, to positions 9 and 10 of anthracene.

The present inventors found that, if radicals which were selected from the group consisting of a phenyl group, a 1-naphthyl group, a 2-naphthyl group, and pyrene were symmetrically or asymmetrically applied to positions 9 and 10 of anthracene and if the radical which was selected from the group consisting of a phenyl group, a 1-naphthyl group, a 2-naphthyl group, and pyrene was applied to position 2 of anthracene as shown in the compound of the following Formula 1, the compound could be used as a light emitting material of the organic light emitting device.

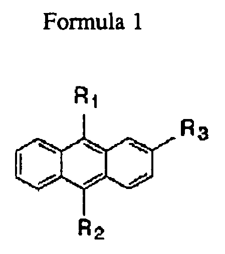

In Formula 1, R1, R2, and R3 are each as defined in the appended Claim 1.

Furthermore, the present inventors found that the organic light emitting device using the compound of Formula 1 has a longer life span than a conventional organic light emitting device using an anthracene derivative which is substituted with aromatic hydrocarbons, and can be driven at a low voltage. Furthermore, they found that, if a predetermined fluorescent material is doped to a light emitting layer comprising the compound of Formula 1, it is possible to significantly increase the driving life of the device.

Examples of the compounds of above-mentioned Formula 1 are compounds of the following Formulae 2 to 14.

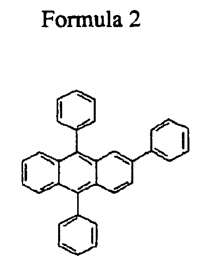

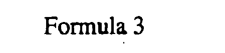

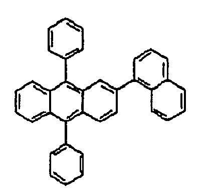

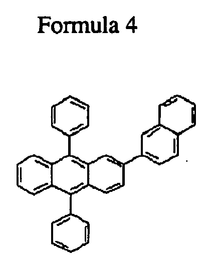

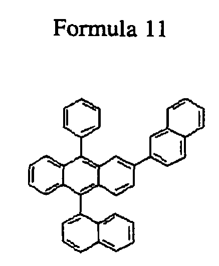

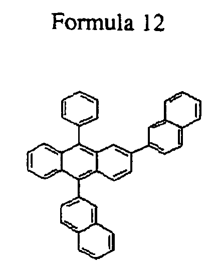

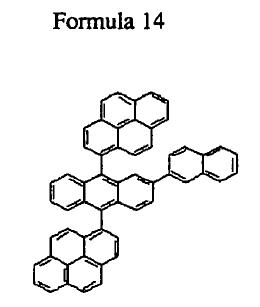

The compounds of the present invention may be produced using starting materials of the following Formula a to Formula c.

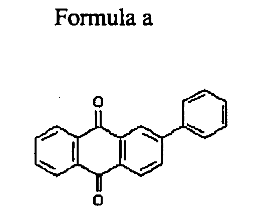

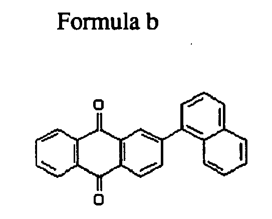

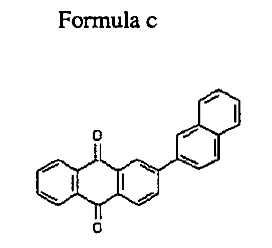

For example. After dissolving 2-bromoanthraquinone completely in toluene, boronic acid, which is selected from the group consisting of phenylboronic acid, 1-naphthaleneboronic acid, and 2-naphthaleneboronic acid, a potassium carbonate solution, tetrakis(triphenylphosphine)palladium(0), and ethanol are added thereto, and reflux is conducted to produce the starting materials of Formula a to Formula c.

Subsequently, t-butyl lithium (5 eq. 1.7 M solution in hexane) and the starting materials of Formula a to Formula c are added to and reacted with a solution of aryl halides in THF at -78°C to produce dialcohols. The Dialcohols, potassium iodide, and sodium hypophosphite are recycled in acetic acid to produce the compound of Formula 1.

A more detailed description of the production will be given in the preparation examples, and it is to be understood that modifications of the methods disclosed in the preparation examples will be apparent to those skilled in the art.

The present invention provides an organic light emitting device which comprises a first electrode, one or more organic material layers, and a second electrode which are sequentially layered. One or more layers of the organic material layers comprise the compound of the above Formula 1.

The organic material layer of the organic light emitting device according to the present invention may have a single layer structure, or alternatively, a multilayered structure in which two or more organic material layers are layered. For example, the organic light emitting device of the present invention may have the organic material layers comprising a hole injection layer, a hole transport layer, a light emitting layer, an electron transport layer, and an electron injection layer. However, the structure of the organic light emitting device is not limited to this, but may comprise a smaller number of organic material layers. Illustrative, but non-limiting examples of the structures of the organic light emitting device according to the present invention are shown in FIGS. 1 to 5.

In the organic light emitting device having the multilayered structure, the compound of Formula 1 may be included in the light emitting layer. Additionally, the layer comprising the compound of Formula 1 may further comprise a light emitting guest material.

In the present invention, illustrative, but non-limiting examples of the light emitting guest material which is capable of being doped in the light emitting layer comprising the compound of the above Formula 1 include the following compounds.

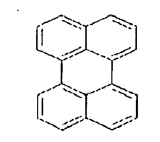

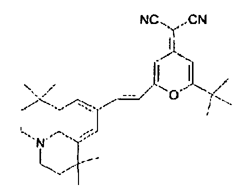

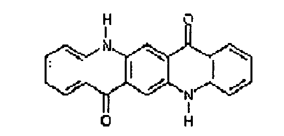

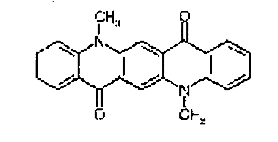

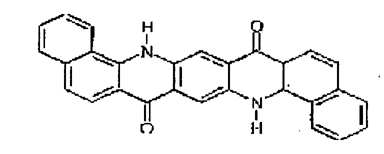

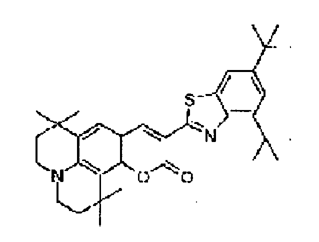

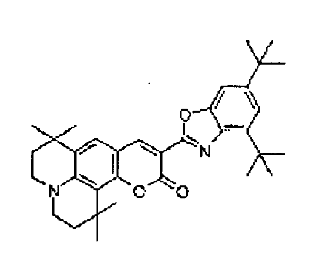

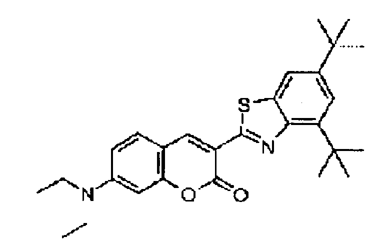

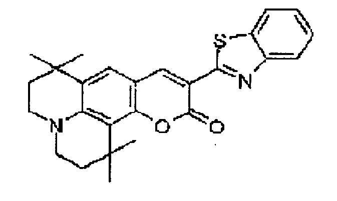

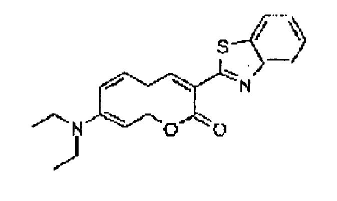

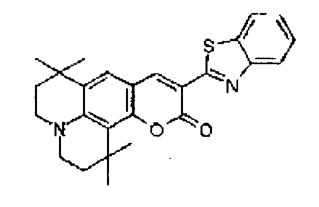

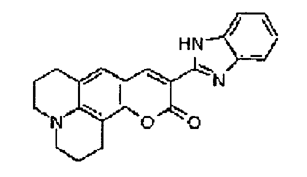

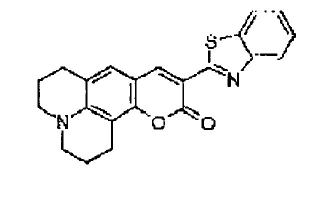

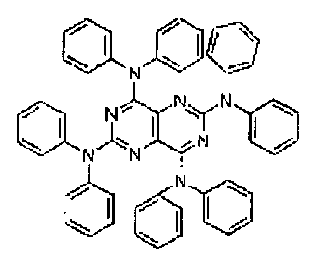

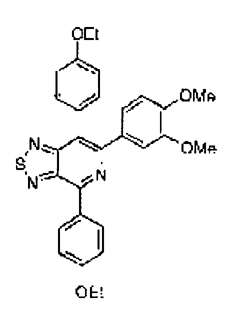

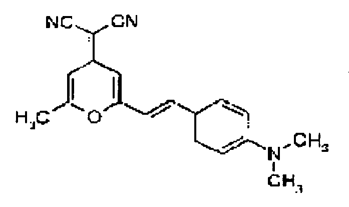

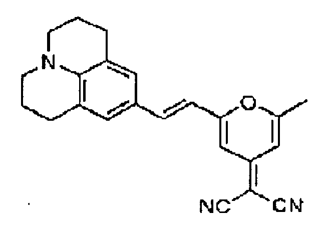

In the present invention, after a dopant as the light emitting guest material is selected, a compound which has a band gap matched with the selected dopant is selected, among the compounds of Formula 1 to be used as a light emitting host.

In the organic light emitting device of the present invention, when a predetermined dopant, for example, the dopant of the following Formula 15, is applied to the light emitting layer containing the compound of Formula 1, it is possible to significantly increase the life of the device.

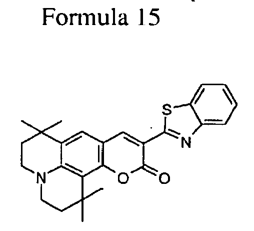

In the organic light emitting device of the present invention, the layer comprising the compound of Formula 1 may be formed between an anode and a cathode through a vacuum deposition method or a solution coating method. Illustrative, but non-limiting examples of the solution coating method are a spin coating method, a dip coating method, a doctor blading method, an inkjet printing method, and a heat transcription method.

The organic light emitting device of the present invention can be produced using known materials and through a known process, except for one or more layers of organic material layers include the compound of Formula 1.

For example, the organic light emitting device of the present invention may be produced by sequentially layering a first electrode, an organic material layer, and a second electrode on a substrate. In connection with this, a physical vapor deposition (PVD) method, such as a sputtering method or an e-beam evaporation method, may be used, but the method is not limited to these.

### Mode for the Invention

A better understanding of the present invention may be obtained in light of the following preparation examples and examples which are set forth to illustrate, but are not to be construed to limit the present invention.

### Preparation of a starting material

### Preparation of a starting material of Formula a

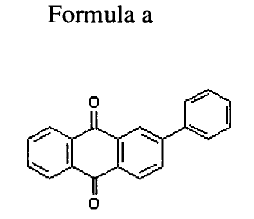

2-bromoanthraquinone (24.2 mmol, 6.96 g) was completely dissolved in 120 mL of toluene, thereto phenylboronic acid (29.0 mmol, 3.54 g), 50 mL of 2M potassium carbonate solution, tetrakis(triphenylphosphine)palladium(0) (0.73 mmol, 0.84 g), and 10 mL of ethanol were added. Reflux was then conducted for 3 hours. After the reaction was completed, it was cooled to room temperature, filtered, and washed a few times using water and ethanol. A filtered solid product was separated using column chromatography and crystallized in ethanol to produce 5.10 g of the compound of Formula a (17.9 mmol, 74 %).

### Preparation of a starting material of Fonnula b

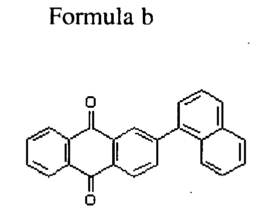

The procedure of producing the starting material of Formula a was repeated to produce 6.5 g of compound of Formula b (19.4 mmol, 80 %) except that 1-naphthaleneboronic acid (29.1 mmol, 5.00 g) was used instead of phenylboronic acid (29.0 mmol, 3.54 g).

### Preparation of a starting material of Formula c

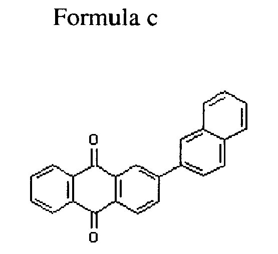

The procedure of producing the starting material of Formula a was repeated to produce 6.5 g of the compound of Formula c (19.4 mmol, 80 %) except that 2-naphthaleneboronic acid (29.1 mmol, 5.00 g) was used instead of phenylboronic acid (29.0 mmol, 3.54 g).

### PREPARATION EXAMPLE 1

### Preparation of a compound of Formula 2

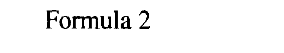

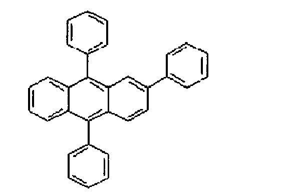

Bromobenzene (8.7 mmol, 1.36 g) was added to 100 mL of dried THF to be completely dissolved therein, and t-butyl lithium (8.5 ml, 1.7 M solution in hexane) was very slowly added thereto at - 78°C. After 1 hour, the starting material of Formula a (2.90 mmol, 0.82 g) was added to the reactants. After 30 min, a cooling vessel was removed, and a reaction was conducted at room temperature for 3 hours. After the reaction was finished, NH₄ Cl aqueous solution was added thereto, and extraction was conducted using ethyl ether. The extract was dried, using anhydrous magnesium sulfate, and concentrated. After a small amount of ethyl ether was added thereto and stirring was conducted, petroleum ether was added and stirring was conducted. Subsequently, filtering and drying were conducted to obtain 1.00 g of dialcohols (2.27 mmol, 78 %).

The resulting dialcohols (1.0 g, 2.27 mmol), potassium iodide (3.77 g, 22.7 mmol), and sodium hypophosphite (4.81 g, 45.4 mmol) were recycled in 200 mL of acetic acid for 3 hours.

The resultant material was cooled to room temperature, filtered, washed a few times using water and methanol, and dried to obtain the compound of Formula 2 (0.85 g, 2.09 mmol, 92 %). MS [M+H] 407.

### PREPARATION EXAMPLE 2

### Preparation of a compound of Formula 3

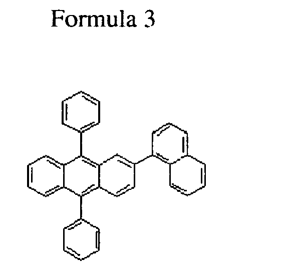

Bromobenzene (8.7 mmol, 1.36 g) was added to 100 mL of dried THF to be completely dissolved therein, and t-butyl lithium (8.5 ml, 1.7 M solution in hexane) was very slowly added thereto at - 78°C. After 1 hour, the starting material of Formula b (2.90 mmol, 0.97 g) was added to the reactants. After 30 min, a cooling vessel was removed, and the reaction was conducted at room temperature for 3 hours. After the reaction was finished, an NH₄Cl aqueous solution was added thereto, and extraction was conducted using ethyl ether. The extract was dried using anhydrous magnesium sulfate and concentrated. A small amount of ethyl ether and petroleum ether were sequentially added thereto, and stirring was conducted for 15 hours. The solid product was filtered and dried to produce 1.30 g of dialcohols (2.65 mmol, 91 %).

The resulting dialcohols (1.30 g, 2.65 mmol), potassium iodide (4.40 g, 26.5 mmol), and sodium hypophosphite (5.60 g, 53.0 mmol) were recycled in 200 mL of acetic acid for 3 hours. Subsequently, the resultant material was cooled to room temperature, filtered, washed a few times using water and methanol, and dried to produce the compound of Formula 3 (1.10 g, 2.41 mmol, 90 %). MS [M+H] 457.

### PREPARATION EXAMPLE 3

### Preparation of a compound of Formula 4

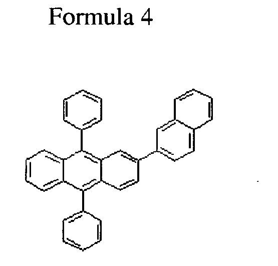

The procedure of preparation example 2 was repeated to produce the compound of Formula 4 (1.10 g, 2.41 mmol, 90 %) except that the starting material of Formula c was used instead of the starting material of Formula b. MS [M+H] 457.

### PREPARATION EXAMPLE 4 (For reference only and outside the scope of the invention)

### Preparation of a compound of Formula 6

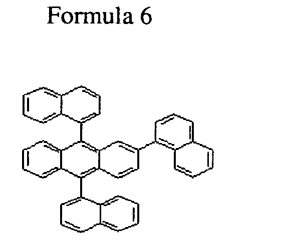

1-bromonaphthalene (8.70 mmol, 1.80 g) was added to 100 mL of dried THF to be completely dissolved therein, and t-butyl lithium (8.5 mL, 1.7 M solution in hexane) was very slowly added thereto at - 78°C. After 1 hour, the starting material of Formula b (2.90 mmol, 0.97 g) was added to the reactants. After 30 min, a cooling vessel was removed, and a reaction was conducted at room temperature for 3 hours. After the reaction was finished, NH₄ Cl aqueous solution was added thereto, and extraction was conducted using ethyl ether. The extract was dried using anhydrous magnesium sulfate and concentrated. A small amount of ethyl ether and petroleum ether were sequentially added thereto, and stirring was conducted for 15 hours. The solid product was filtered and dried to produce 1.50 g of dialcohols (2.54 mmol, 88 %).

The resulting dialcohols (1.50 g, 2.54 mmol), potassium iodide (4.21 g, 25.4 mmol), and sodium hypophosphite (5.38 g, 50.8 mmol) were recycled in 200 mL of acetic acid for 3 hours. The resultant material was cooled to room temperature, filtered, washed a few times using water and methanol, and dried to produce the compound of Formula 6 (1.30 g, 2.34 mmol, 92 %). MS [M+H] 557.

### PREPARATION EXAMPLE 5 (For reference only and outside the scope of the invention)

### Preparation of a compound of Formula 8

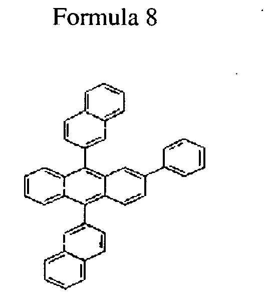

2-bromonaphthalene (8.70 mmol, 1.80 g) was added to 100 mL of dried THF to be completely dissolved therein, and t-butyl lithium (8.5 mL, 1.7 M solution in hexane) was very slowly added thereto at - 78°C. After 1 hour, the starting material of Formula a (2.90 mmol, 0.82 g) was added to the reactants. After 30 min, a cooling vessel was removed, and the reaction was conducted at room temperature for 3 hours. After the reaction was finished, an NH₄Cl aqueous solution was added thereto, and extraction was conducted using ethyl ether. The extract was dried using anhydrous magnesium sulfate and concentrated. A small amount of ethyl ether and petroleum ether were sequentially added thereto, and stirring was conducted for 15 hours. The solid product was filtered and dried to produce 1.40 g of dialcohols (2.58 mmol, 89 %).

The resulting dialcohols (1.40 g, 2.58 mmol), potassium iodide (4.28 g, 25.8 mmol), and sodium hypophosphite (5.46 g, 51.6 mmol) were recycled in 200 mL of acetic acid for 3 hours. The resultant material was cooled to room temperature, filtered, washed a few times using water and methanol, and dried to produce the compound of Formula 8 (1.20 g, 2.37 mmol, 92 %). MS [M+H] 507.

### PREPARATION EXAMPLE 6 (For reference only and outside the scope of the invention)

### Preparation of a compound of Formula 9

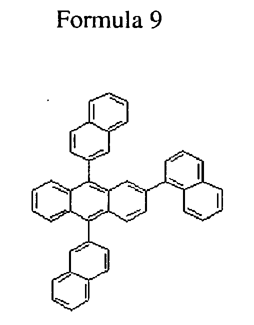

The procedure of preparation example 4 was repeated to produce the compound of Formula 9 (1.30 g, 2.34 mmol, 92 %) except that 2-bromonaphthalene was used instead of 1-bromonaphthalene. MS [M+H] 557.

### PREPARATION EXAMPLE 7 (For reference only and outside the scope of the invention)

### Preparation of a compound of Formula 10

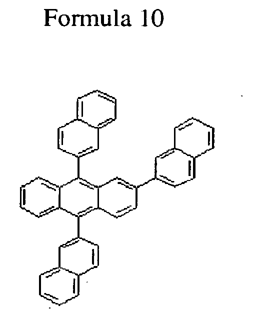

The procedure of preparation example 6 was repeated to produce the compound of Formula 10 (1.30 g, 2.34 mmol, 92 %) except that the starting material of Formula c was used instead of the starting material of Formula b. MS [M+H] 557.

### PREPARATION EXAMPLE 8

### Preparation of a compound of Formula 12

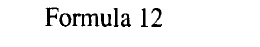

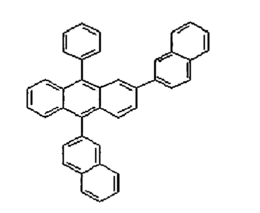

Bromobenzene (9.87 mmol, 1.55 g) was added to 100 mL of dried THF to be completely dissolved therein, and t-butyl lithium (6.9 mL, 1.7 M solution in hexane) was very slowly added thereto at - 78°C. After 1 hour, the starting material of Formula c (8.97 mmol, 3.00 g) was added to the reactants. After 30 min, a cooling vessel was removed, and a reaction was conducted at room temperature for 3 hours. After the reaction was finished, an NH₄Cl aqueous solution was added thereto, and extraction was conducted using ethyl ether. The extract was dried using anhydrous magnesium sulfate, concentrated, separated using column chromatography, and dried to produce 1.40 g of alcohols (3.39 mmol, 38 %).

2-bromonaphthalene (5.90 mmol, 1.22 g) was added to 50 mL of dried THF to be completely dissolved therein, and t-butyl lithium (5 mL, 1.7 M solution in hexane) was very slowly added thereto at - 78°C. After 1 hour, alcohols as described above (1.69 mmol, 0.70 g) were added to the reactants. After 30 min, a cooling vessel was removed, and a reaction was conducted at room temperature for 3 hours. After the reaction was finished, NH₄Cl aqueous solution was added thereto, and extraction was conducted using ethyl ether. A small amount of ethyl ether and petroleum ether were sequentially added thereto, and stirring was conducted for 15 hours. The solid product was filtered and dried to produce 0.64 g of dialcohols (1.18 mmol, 70 %).

The resulting dialcohols (0.64 g, 1.18 mmol), potassium iodide (1.97 g, 11.84 mmol), and sodium hypophosphite (2.50 g, 23.68 mmol) were recycled in 100 mL of acetic acid for 3 hours. The resultant material was cooled to room temperature, filtered, washed a few times using water and methanol, and dried to produce the compound of Formula 12 (0.50 g, 0.99 mmol, 84 %). MS [M+H] 507.

### PREPARATION EXAMPLE 9

### Preparation of a compound of Formula 14

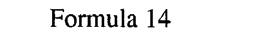

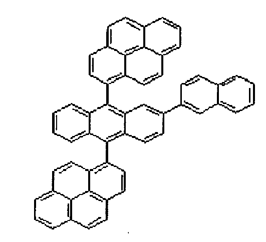

1-bromopyrene (8.70 mmol, 2.5 g) was added to 100 mL of dried THF to be completely dissolved therein, and t-butyl lithium (8.5 mL, 1.7 M solution in hexane) was very slowly added thereto at - 78°C. After 1 hour, the starting material of Formula c (2.90 mmol, 1.0 g) was added to the reactants. After 30 min, a cooling vessel was removed, and a reaction was conducted at room temperature for 3 hours. After the reaction was finished, NH₄ Cl aqueous solution was added thereto, and extraction was conducted using ethyl ether. The extract was dried using anhydrous magnesium sulfate and concentrated. A small amount of ethyl ether and petroleum ether were sequentially added thereto, and stirring was conducted for 15 hours. The solid product was filtered and dried to produce 1.93 g of dialcohols (2.61 mmol, 90 %).

Dialcohols (1.93 g, 2.61 mmol), potassium iodide (4.33 g, 26.1 mmol), and sodium hypophosphite (5.53 g, 52.2 mmol) were recycled in 200 mL of acetic acid for 3 hours. The resultant material was cooled to room temperature, filtered, washed a few times using water and methanol, and dried to produce the compound of Formula 14 (1.69 g, 2.4 mmol, 92 %). MS [M+H] 704.

### Preparation of an organic light emitting device

### EXAMPLE 1

A glass substrate, on which ITO (indium tin oxide) was applied to a thickness of 1500 Å to form a thin film, was put in distilled water, in which a detergent was dissolved, and then washed using ultrasonic waves. A product manufactured by Fischer Inc. was used as a detergent, and distilled water was produced through filtering twice using a filter manufactured by Millipore Inc. After ITO was washed for 30 min, ultrasonic washing was conducted in distilled water twice for 10 min. After the washing using distilled water was finished, ultrasonic washing was conducted using a solvent, such as isopropyl alcohol, acetone, or methanol, and drying was then conducted. Subsequently, it was transported to a plasma washing machine. The substrate was washed using oxygen plasma for 5 min, and then transported to a vacuum evaporator.

Hexanitrile hexaazatriphenylene of the following Formula was vacuum deposited to a thickness of 500 Å by heating on a transparent ITO electrode, which was prepared through the above procedure, to form a hole injection layer.

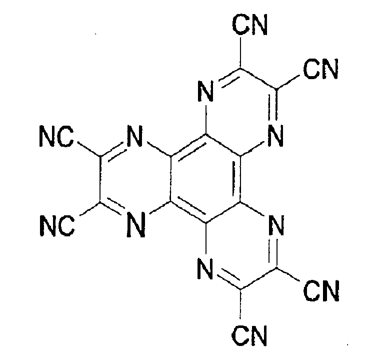

Subsequently, NPB (400 Å) which was a material for transporting holes was vacuum deposited on the hole injection layer, and the compound of Formula 4 produced in preparation example 3 was vacuum deposited thereonto in a thickness of 300 Å to form a light emitting layer. A compound of the following Formula for injecting and transporting electrons was vacuum deposited on the light emitting layer to a thickness of 200 Å.

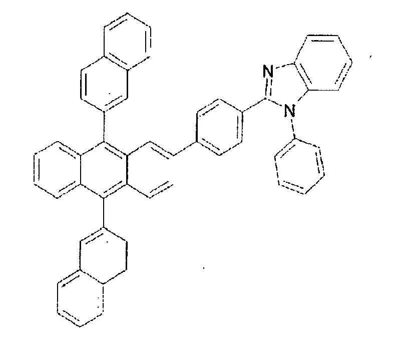

Lithium fluoride (LiF) and aluminum were sequentially deposited to thicknesses of 5 Å and 2500 Å, respectively, on the electron injection and transport layer to form a cathode, thereby the organic light emitting device was created.

In the above procedure, the deposition speed of an organic material was maintained at 1 Å/sec, and lithium fluoride and aluminum were deposited at a speed of 0.2 Å/sec and 3 - 7 Å/sec, respectively.

A forward electric field of 6.2 V was applied to the resulting organic light emitting device, and a blue spectrum having brightness of 1400 nit was observed at a current density of 100 mA/cm², and corresponded to x = 0.18 and y = 0.23 based on a 1931 CIE color coordinate. Furthermore, when a constant direct current was applied to the device at a current density of 50 mA/cm², the time required to reduce brightness to 50 % of initial brightness was 600 hours.

### EXAMPLE 2 For reference only and outside the scope of the invention

An organic light emitting device was produced through the same procedure as example 1 except that a light emitting layer was formed using the compound of Formula 9 produced in preparation example 6 instead of the compound of Formula 4.

A forward electric field of 6.7 V was applied to the resulting organic light emitting device, and a blue spectrum having brightness of 1380 nit, which corresponds to x = 0.17 and y = 0.22 based on a 1931 CIE color coordinate, was observed at a current density of 100 mA/cm². Furthermore, when a constant direct current was applied to the device at a current density of 50 mA/cm², the time required to reduce brightness to 50 % of initial brightness was 500 hours.

### EXAMPLE 3 For reference only and outside the scope of the invention

An organic light emitting device was produced through the same procedure as example 1, except that the light emitting layer was formed using the compound of Formula 10 produced in preparation example 7 instead of the compound of Formula 4.

A forward electric field of 6.5 V was applied to the resulting organic light emitting device, and a blue spectrum having brightness of 1410 nit, which corresponds to x = 0.17 and y = 0.22 based on a 1931 CIE color coordinate, was observed at a current density of 100 mA/cm². Furthermore, when a constant direct current was applied to the device at a current density of 50 mA/cm², the time required to reduce brightness to 50 % of initial brightness was 450 hours.

### EXAMPLE 4

An organic light emitting device was produced through the same procedure as example 1 except that the light emitting layer was formed using the compound of Formula 12 produced in preparation example 8 instead of the compound of Formula 4.

A forward electric field of 6.3 V was applied to the resulting organic light emitting device, and a blue spectrum having brightness of 1500 nit, which corresponds to x = 0.17 and y = 0.21 based on a 1931 CIE color coordinate, was observed at a current density of 100 mA/cm**².** Furthermore, when a constant direct current was applied to the device at a current density of 50 mA/cm², the time required to reduce brightness to 50 % of initial brightness was 260 hours.

### EXAMPLE 5

An organic light emitting device was produced through the same procedure as example 1 except that the compound of Formula 15 was doped to the compound of Formula 4 produced in preparation example 3 at a ratio of 100:2 to form the light emitting layer to a thickness of 300 Å instead of forming the light emitting layer using only the compound of Formula 4.

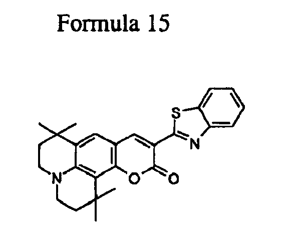

A forward electric field of 6.0 V was applied to the resulting organic light emitting device, and a green spectrum having brightness of 7200 nit, which corresponds to x = 0.232 and y = 0.618 based on a 1931 CIE color coordinate, was observed at a current density of 100 mA/cm². Furthermore, when a constant direct current was applied to the device at a current density of 50 mA/cm², the time required to reduce brightness to 50 % of initial brightness was 1250 hours.

### EXAMPLE 6 For reference only and outside the scope of the invention

An organic light emitting device was produced through the same procedure as example 5 except that the light emitting layer was formed using the compound of Formula 9 produced in preparation example 6 instead of using the compound of Formula 4.

A forward electric field of 6.1 V was applied to the resulting organic light emitting device, and a green spectrum having brightness of 7100 nit, which corresponds to x = 0.231 and y = 0.617 based on a 1931 CIE color coordinate, was observed at a current density of 100 mA/cm². Furthermore, when a constant direct current was applied to the device at a current density of 50 mA/cm², the time required to reduce brightness to 50 % of initial brightness was 1200 hours.

### EXAMPLE 7 For reference only and outside the scope of the invention

An organic light emitting device was produced through the same procedure as example 5 except that the light emitting layer was formed using the compound of Formula 10, produced in preparation example 7, instead of using the compound of Formula 4.

A forward electric field of 6.2 V was applied to the resulting organic light emitting device, and a green spectrum having brightness of 7000 nit, which corresponds to x = 0.231 and y = 0.618 based on a 1931 CIE color coordinate, was observed at a current density of 100 mA/cm². Furthermore, when a constant direct current was applied to the device at a current density of 50 mA/cm², the time required to reduce brightness to 50 % of initial brightness was 1200 hours.

### EXAMPLE 8

An organic light emitting device was produced through the same procedure as example 5 except that the light emitting layer was formed using the compound of Formula 12, produced in preparation example 8, instead of using the compound of Formula 4.

A forward electric field of 6.0 V was applied to the resulting organic light emitting device, and a green spectrum having brightness of 7900 nit, which corresponds to x = 0.249 and y = 0.617 based on a 1931 CIE color coordinate, was observed at a current density of 100 mA/cm². Furthermore, when a constant direct current was applied to the device at a current density of 50 mA/cm², the time required to reduce brightness to 50 % of initial brightness was 800 hours.

### EXAMPLE 9

An organic light emitting device was produced through the same procedure as example 1 except that the light emitting layer was formed using the compound of Formula 14 produced in preparation example 9 instead of using the compound of Formula 4.

A forward electric field of 7.25 V was applied to the resulting organic light emitting device, and a green spectrum having brightness of 2640 nit, which corresponds to x = 0.44 and y = 0.36 based on a 1931 CIE color coordinate, was observed at a current density of 100 mA/cm². Furthermore, when a constant direct current was applied to the device at a current density of 50 mA/cm², the time required to reduce brightness to 50 % of initial brightness was 300 hours.

### COMPARATIVE EXAMPLE 1

An organic light emitting device was produced through the same procedure as example 5 except that the light emitting layer was formed using a compound of the following Formula, disclosed in US Patent No. 5,935,721, instead of using the compound of Formula 4.

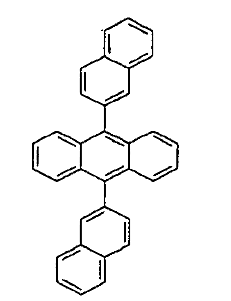

A forward electric field of 6.6 V was applied to the resulting organic light emitting device, and a green spectrum having brightness of 7000 nit, which corresponds to x = 0.272 and y = 0.61 based on a 1931 CIE color coordinate, was observed at a current density of 100 mA/cm². Furthermore, when a constant direct current was applied to the device at a current density of 50 mA/cm², the time required to reduce brightness to 50 % of initial brightness was 300 hours.

Materials of the light emitting layer, which were used in examples and the comparative example, along with test results, are summarized in the following Table 1.

**Table 1**

| | Material of light emitting layer | Driving voltage( V) | Current density (mA/cm² ) | Color coordinate* | brightness (nit) | Life span** (hours) |
|---|---|---|---|---|---|---|
| Example 1 | Formula 4 | 6.2 | 100 | x=0.18y=0. 23 | 1400 | 200 |
| Example 2⁺ | Formula 9 | 6.7 | 100 | x=0.17y=0. 22 | 1380 | 500 |
| Example 3⁺ | Formula 10 | 6.5 | 100 | x=0.17y=0. 22 | 1410 | 450 |
| Example 4 | Formula 12 | 6.3 | 100 | x=0.17y=0. 21 | 1500 | 260 |
| Example 5 | Formula 4,doping of green light emitting material *** | 6.0 | 100 | x=0.232y=0 .618 | 7200 | 1250 |
| Example 6⁺ | Formula 9,doping of green light emitting material *** | 6.1 | 100 | x=0.231y=0 .617 | 7100 | 1200 |
| Example 7⁺ | Formula 10,doping of green light emitting material *** | 6.2 | 100 | x=0.231y=0 .618 | 7000 | 1200 |
| Example 8 | Formula 12,doping of green light emitting material *** | 6.0 | 100 | x=0.249y=0 .617 | 7900 | 800 |
| Example 9 | Formula 14 | 7.25 | 50 | x=0.44y=0. 36 | 2640 | 300 |
| Comparati ve Example 1 | 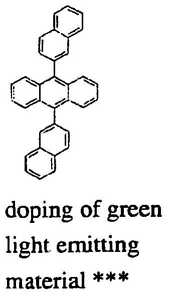 | 6.6 | 100 | x=0.272y=0 .610 | 7000 | 300 |
| Color coordinate*: based on 1931 CIE color coordinateLife span**: A time which is required to reduce brightness to 50 % of initial brightness when a constant direct current is applied at a current density of 50 mA/cm²Green light emitting material***: 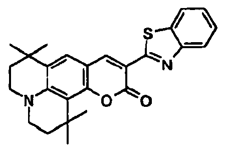 | | | | | | |

| | | | | | | |
|---|---|---|---|---|---|---|
| ⁺(For reference only and outside the scope of the invention) | | | | | | |

From the above Table 1, it can be seen that, when the compound of the present invention is used as the sole light emitting material or light emitting host in the organic light emitting device, a life span of the device is significantly improved and it is possible to drive the device at a low voltage in comparison with a conventional anthracene derivative which is substituted with aromatic hydrocarbons.

### Industrial Applicability

A compound of the present invention can be used as a sole light emitting material or light emitting host of an organic light emitting device, and the organic light emitting device using the compound of the present invention has a long life span and can be driven at a low voltage.

## Claims

1. A compound having the following Formula 1: 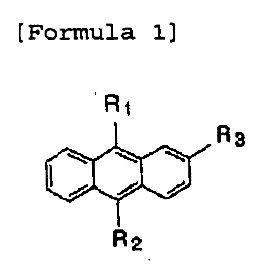 wherein:
R₁ is selected from the group consisting of a phenyl group and a pyrene, and
R₂ and R₃ each independently is selected from the group consisting of a phenyl group, an 1-naphthyl group, a 2-naphthyl group, and a pyrene.

2. The compound as set forth in claim 1, wherein the compound of Formula 1 is selected from a group consisting of compounds of the following Formulae 2 to 4, 11, 12 and 14 :

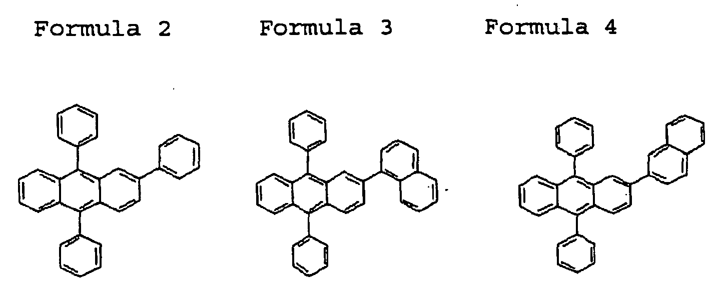

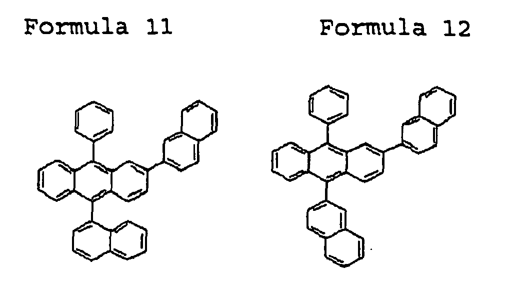

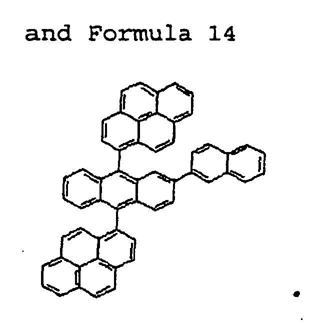

3. An organic light emitting device comprising a first electrode, one or more organic material layers, and a second electrode which are sequentially layered, wherein one or more layers of the organic material layers include a compound of Formula 1: 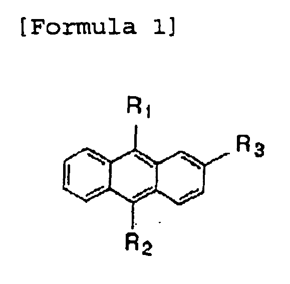 wherein:
R₁ is selected from the group consisting of a phenyl group and a pyrene, and
R₂ and R₃ each independently is selected from the group consisting of a phenyl group, an 1-naphthyl group, a 2-naphthyl group, and a pyrene.

4. The organic light emitting device as set forth in claim 3, wherein said organic material layers comprise light emitting layers, and the light emitting layercomprises the compound of Formula 1.

5. The organic light emitting device as set forth in claim 3, wherein the organic material layer, which includes the compound of Formula 1, further comprises a light emitting guest material.

6. The organic light emitting device as set forth in claim 5, wherein said light emitting guest material is a compound with the following Formula 15:

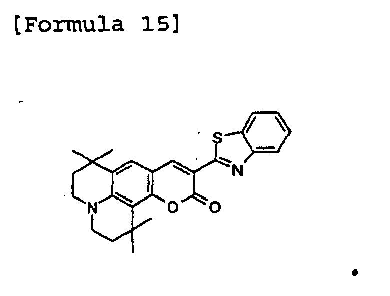

## Patentansprüche

1. Verbindung der folgenden Formel (1): 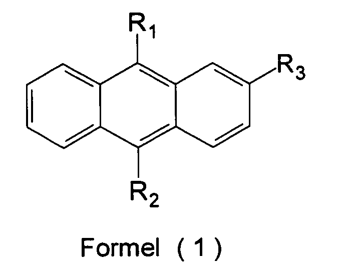 worin:
R₁ aus der Gruppe bestehend aus einer Phenylgruppe und einem Pyren ausgewählt ist, und
R₂ und R₃ jeweils unabhängig voneinander aus der Gruppe bestehend aus einer Phenylgruppe, einer 1-Naphthylgruppe, einer 2-Naphthylgruppe und einem Pyren ausgewählt sind.

2. Verbindung, wie in Anspruch 1 dargelegt, wobei die Verbindung der Formel (1) aus der Gruppe bestehend aus Verbindungen der folgenden Formeln (2) bis (4), (11), (12) und (14) ausgewählt ist:

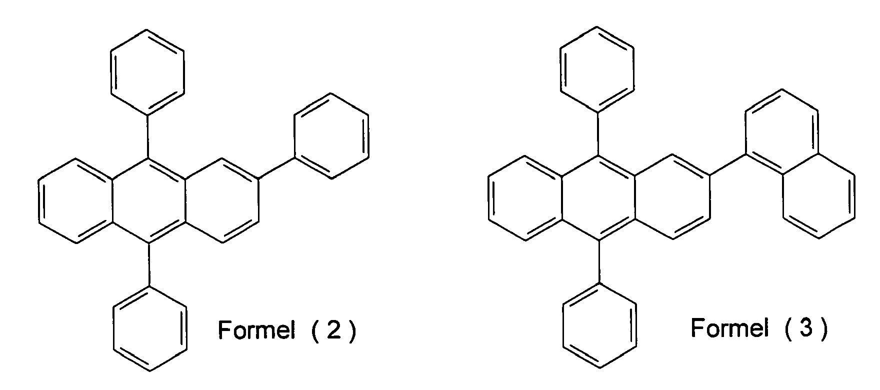

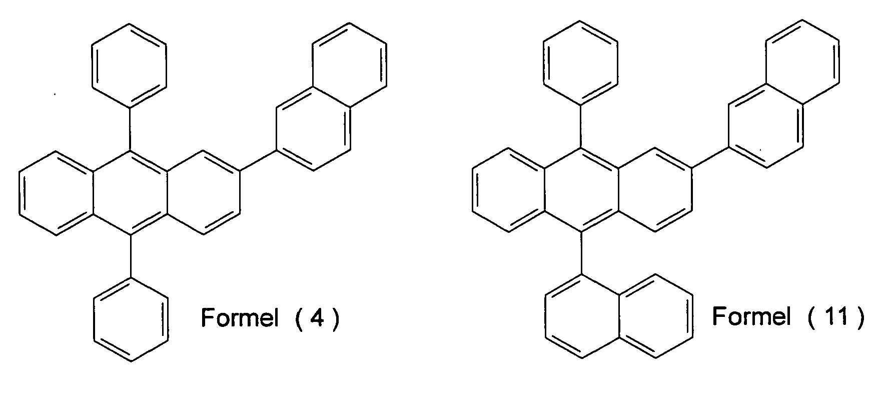

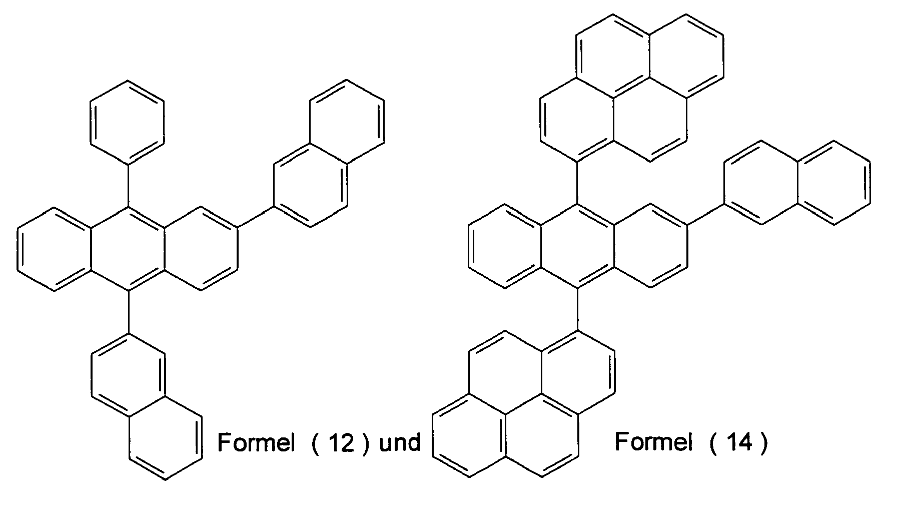

3. Organische lichtemittierende Vorrichtung, umfassend eine erste Elektrode, eine oder mehrere organische Materialschicht(en) und eine zweite Elektrode, die der Reihe nach überlagert sind, worin eine oder mehrere Schicht(en) der organischen Materialschichten eine Verbindung der Formel (1) einschliess(t/en): 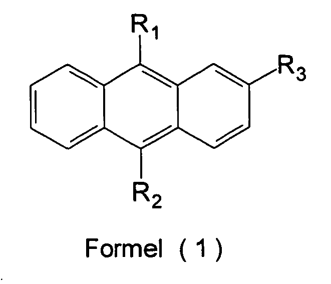 worin:
R₁ aus der Gruppe bestehend aus einer Phenylgruppe und einem Pyren ausgewählt ist, und
R₂ und R₃ jeweils unabhängig voneinander aus der Gruppe bestehend aus einer Phenylgruppe, einer 1-Naphthylgruppe, einer 2-Naphthylgruppe und einem Pyren ausgewählt sind.

4. Organische lichtemittierende Vorrichtung, wie in Anspruch 3 dargelegt, wobei die organischen Materialschichten lichtemittierende Schichten umfassen und die lichtemittierenden Schichten die Verbindung der Formel (1) umfassen.

5. Organische lichtemittierende Vorrichtung, wie in Anspruch 3 dargelegt, wobei die organischen Materialschichten, die die Verbindung der Formel (1) einschliessen, ferner ein lichtemittierendes Gastmaterial umfassen.

6. Organische lichtemittierende Vorrichtung, wie in Anspruch 5 dargelegt, wobei das lichtemittierende Gastmaterial eine Verbindung der folgenden Formel (15) ist:

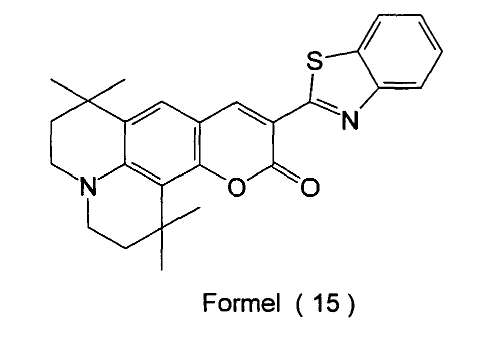

## Revendications

1. Composé ayant la Formule 1 suivants : 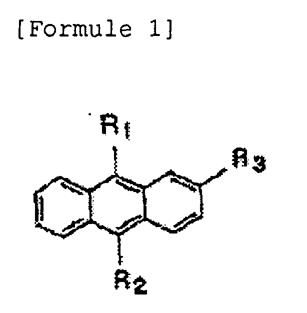 dans laquelle :
R₁ est choisi parmi le groupe consistant en un groupe phényle et un pyrène, et
R₂ et R₃ chacun indépendamment est choisi parmi le groupe consistant en un groupe phényle, un groupe 1-naphtyle, un groupe 2-naphtyle, et un pyrène.

2. Composé selon la revendication 1, dans lequel le composé de Formule 1 est choisi parmi un groupe consistant en des composés des Formules 2 à 4, 11, 12 et 14 suivantes :

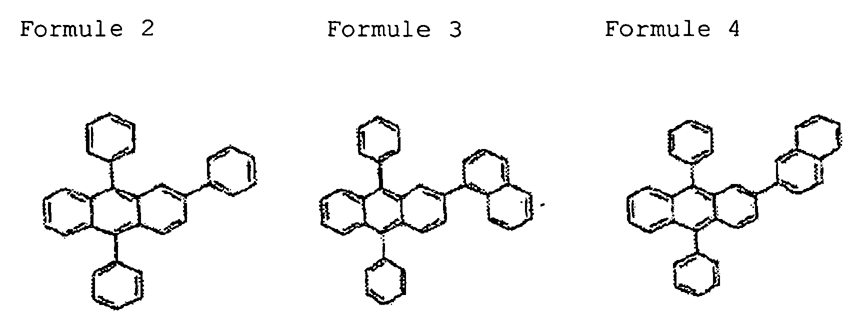

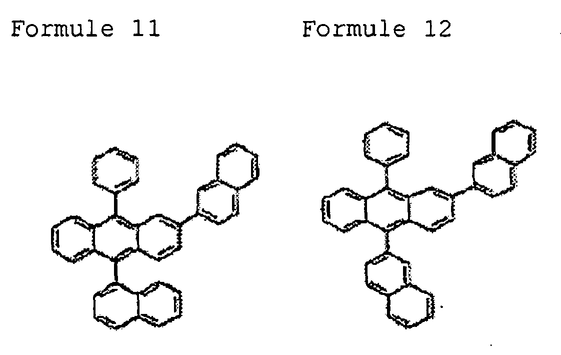

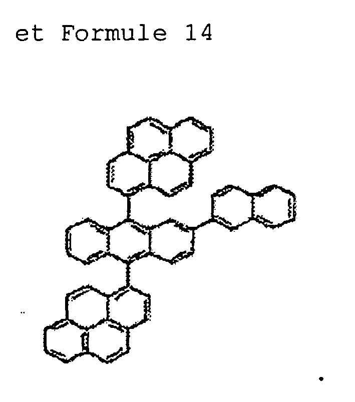

3. Dispositif organique électroluminescent comprenant une première électrode, une ou plusieurs couches d'une matière organique, et une deuxième électrode qui sont déposées séquentiellement en couches, dans lequel une ou plusieurs couches des couches d'une matière organique inclut un composé de Formule 1 : 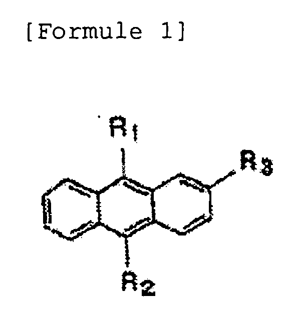 dans laquelle :
R₁ est choisi parmi le groupe consistant en un groupe phényle et un pyrène, et
R₂ et R₃ chacun indépendamment est choisi parmi le groupe consistant en un groupe phényle, un groupe 1-naphtyle, un groupe 2-naphtyle, et un pyrène.

4. Dispositif organique électroluminescent selon la revendication 3, dans lequel lesdites couches d'une matière organique comprennent des couches électroluminescentes, et la couche électroluminescente comprend le composé de Formule 1.

5. Dispositif organique électroluminescent selon la revendication 3, dans lequel la couche d'une matière organique, qui inclut le composé de Formule 1, comprend en outre une matière incluse électroluminescente.

6. Dispositif organique électroluminescent selon la revendication 5, dans lequel ladite matière incluse électroluminescente est un composé avec la Formule 15 suivants :
